# EUROPÄISCHE PATENTANMELDUNG

(11) **EP 0 747 690 A1**
(43) Veröffentlichungstag der Anmeldung: **11.12.1996**
(21) Anmeldenummer: 96105871.6
(22) Anmeldetag: 15.04.1996
(51) Int. Cl.: G01N 1/26, B08B 9/46

(54) **Vorrichtung zur Inspektion von Behältern**

(30) Priorität: 08.06.1995 CH 1679/95
(71) Anmelder: ELPATRONIC AG, CH-6303 Zug (CH)
(72) Erfinder: Gysi, Peter, 5454 Bellikon (CH)

(57) **Zusammenfassung**

Oberhalb eines linearen Förderwegabschnittes (1) mit kontinuierlicher Förderung von Flaschen ist ein scheibenförmiges, vertikal angeordnetes Probeentnahmeorgan (5) angeordnet, welches synchron zur Förderung der Behälter dreht. Dies ergibt eine besonders einfache und kostengünstige Vorrichtung.

## Beschreibung

Die Erfindung betrifft eine Vorrichtung zur Inspektion von Behältern, insbesondere von Mehrweg-Flaschen, auf das Vorhandensein von Verunreinigungen, umfassend eine Fördereinrichtung für die Behälter und mindestens ein jeweils aus einem Behälter eine Probe entnehmendes Probeentnahmeorgan.

Bekannte Vorrichtungen mit hohem Flaschendurchsatz verwenden eine Karussellfördereinrichtung, so z.B. die Vorrichtung gemäss EP-A-0 579 952,DE-U-91 14 357 oder EP-A-0 534 096. Eine solche Fördereinrichtung hat den Nachteil, dass sie relativ kompliziert und kostenaufwendig ist. Ferner benötigt eine solche Einrichtung viel Platz. Aus der US-A-3 266 292 hingegen ist eine Inspektionsvorrichtung mit einem linearen Förderweg bekannt. Dort erfolgt aber jedesmal ein Anhalten des Behälters zur Einführung des Probeentnahmeorgans, was eine solche Vorrichtung untauglich für die heutige industrielle Inspektion von Behältern macht, wo z.B. bis zu 1000 Behälter pro Minute geprüft werden müssen, da der verlangte Behälterdurchsatz durch die Vorrichtung nicht erreicht werden kann.

Der Erfindung liegt die Aufgabe zugrunde, eine Inspektionsvorrichtung zu schaffen, welche bei einfachem und kostengünstigen Aufbau einen hohen Behälterdurchsatz pro Zeiteinheit erzielen kann und die erwähnten Nachteile nicht aufweist.

Diese Aufgabe wird bei einer Inspektionsvorrichtung der eingangs genannten Art dadurch erreicht, dass im Inspektionsbereich ein linearer Förderweg zur kontinuierlichen Behälterförderung vorgesehen ist, oberhalb dessen ein parallel zum Förderweg angeordnetes, rad- oder scheibenförmiges Probeentnahmeorgan vorgesehen ist, das um eine quer zum Förderweg liegende Achse drehend antreibbar ist.

Dadurch, dass ein linearer Förderweg mit kontinuierlicher Förderung vorgesehen ist, ergibt sich bei einfachem und kostengünstigen Aufbau die Möglichkeit eines hohen Behälterdurchsatzes. Das oberhalb des Förderwegs und parallel dazu angeordnete rad- oder scheibenförmige Probeentnahmeorgan erlaubt die Entnahme der Proben bei kontinuierlicher linearer Förderung und damit ebenfalls den hohen Behälterdurchsatz pro Zeiteinheit. Im Vergleich mit den bekannten Karussellvorrichtungen können bei der Probeentnahme zudem sehr kurze Gaswege realisiert werden.

Im folgenden werden Ausführungsbeispiele der Erfindung anhand der Zeichnungen näher erläutert. Dabei zeigt
Figur 1 schematisch die Ansicht einer Vorrichtung gemäss der Erfindung;
Figur 1a schematisch die Anordnung zweier Vorrichtungen nach Figur 1;
Figur 2 schematisch eine Ansicht des Probeentnahmeorgans oberhalb des linearen Förderweges mit den Behältern;
Figur 3 eine Frontalansicht eines Probeentnahmeorganes;
Figur 4 einen Vertikalschnitt durch das Probeentnahmeorgan nach Figur 3;
Figur 4a eine alternative Ausgestaltung der Probegaskanäle in der Ausführung gemäss Figur 4;
Figur 5 eine weitere Frontalansicht eines Probeentnahmeorgans;
Figur 6 einen nur teilweise dargestellten Vertikalschnitt durch das Probeentnahmeorgan nach Figur 5 in einer ersten Stellung;
Figur 7 einen ebenfalls nur teilweise dargestellten Vertikalschnitt durch das Probeentnahmeorgan von Figur 5;
Figur 8 eine teilweise Ansicht eines weiteren Probeentnahmeorgans, und
Figur 9 eine weitere Ansicht eines Probeentnahmeorgans.

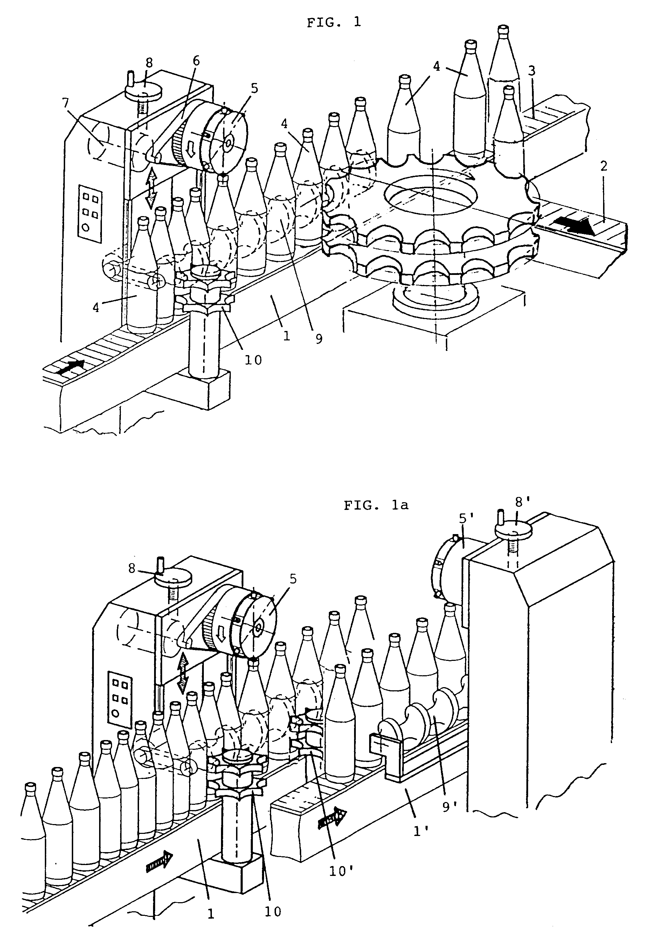

Figur 1 zeigt schematisch eine Vorrichtung gemäss der Erfindung mit einem linearen Förderwegabschnitt 1, an den sich nach der Vorrichtung zwei sich verzweigende Förderwege 2,3 anschliessen. Oberhalb der auf dem Förderwegabschnitt 1 kontinuierlich geförderten Behälter 4 befindet sich das drehend angetriebene Probeentnahmeorgan 5, welches im dargestellten Beispiel mittels eines Zahnriemens 6 durch einen Motor 7 angetrieben wird. Das Probeentnahmeorgan 5 ist in seiner Höhe über dem Förderwegabschnitt 1 einstellbar, was in Figur 1 mit der Kurbel 8 symbolisiert ist, um die Höhenlage des Organs 5 an die Behälterhöhe anzupassen. Im Bereich des linearen Förderwegabschnittes 1 erfolgt vorzugsweise eine genaue und zwangsgeführte Positionierung der Behälter 4, was z.B. mittels der in gestrichelten Linien dargestellten Schnecke 9 erfolgt. Am Anfang des linearen Abschnittes 1 kann ein Stern 10 vorgesehen sein, welcher ein geordnetes Eintreten der Behälter 4 in die Schnecke 9 sicherstellt und zudem als Sperrorgan dient. Durch die genaue Positionierung der Behälter 4 kann eine zwangssynchrone Bewegung der Behälter und des Probeentnahmeorgans 5 sichergestellt werden. Zur Ausleitung der bei der Inspektion als mangelhaft erkannten Behälter kann ein Ausleitmodul mit sich verzweigenden Förderwegen 2,3 vorgesehen sein, so dass z.B. als gut erkannte Flaschen in den Förderweg 2 geleitet werden, als schlecht erkannte Flaschen in den Förderweg 3. Natürlich können auch andere bekannte Selektionsmöglichkeiten verwendet werden, z.B. Auswerfer oder Weichen.

Der gezeigte einfache Aufbau der Vorrichtung ermöglicht eine problemlose Aufstellung entlang einer Förderlinie; es können auch zwei oder mehrere solcher Vorrichtungen in Serie geschaltet sein. Bei parallelen Förderlinien können auf einfache Weise zwei Vorrichtungen angeordnet sein, wie dies aus Figur 1a ersichtlich ist.

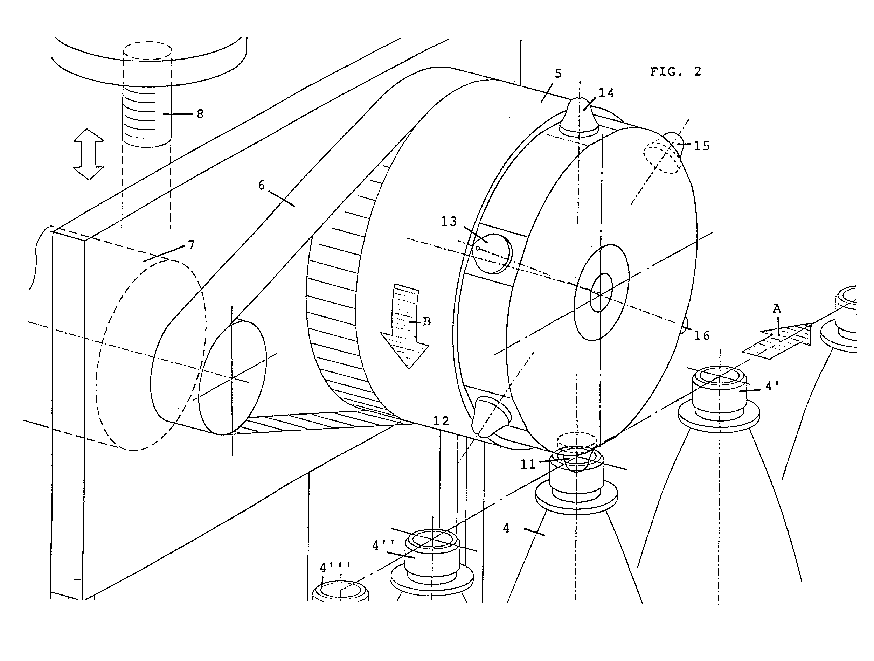

Figur 2 zeigt grob schematisch eine Ansicht des angetriebenen Probeentnahmeorgans 5 und dessen Eintauchen in die jeweiligen Behälter. Dazu ist im gezeigten Beispiel das Probeentnahmeorgan 5 mit sechs Entnahmevorsprüngen 11 bis 16 ausgestattet, welche bei der Drehung des Probeentnahmeorgans 5, welches im gezeigten Beispiel scheibenförmig ist und die Vorsprünge 11-16 an seinem Scheibenumfang trägt, in die Abfolge der Behälter 4 nacheinander eintauchen. In der dargestellten Stellung taucht also der Vorsprung 11 in den Behälter 4 ein, während der Vorsprung 16 den Behälter 4' bereits verlassen hat. Bei der linearen Förderung der Behälter in Richtung des Pfeiles A und der Drehbewegung des Organs 5 in Richtung des Pfeiles B, welche Bewegungen miteinander synchronisiert sind, ergibt sich als nächstes ein Eintauchen des Vorsprunges 12 in den Behälter 4'' während der Vorsprung 11 den Behälter 4 wieder verlässt. Als nächstes erfolgt dann ein Eintauchen des Vorsprunges 13 in den Behälter 4''' usw.

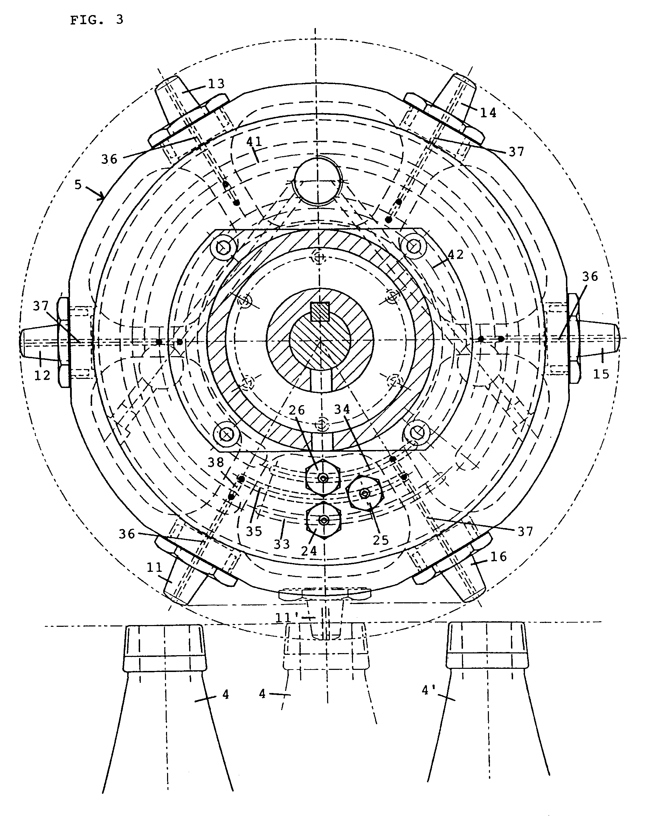

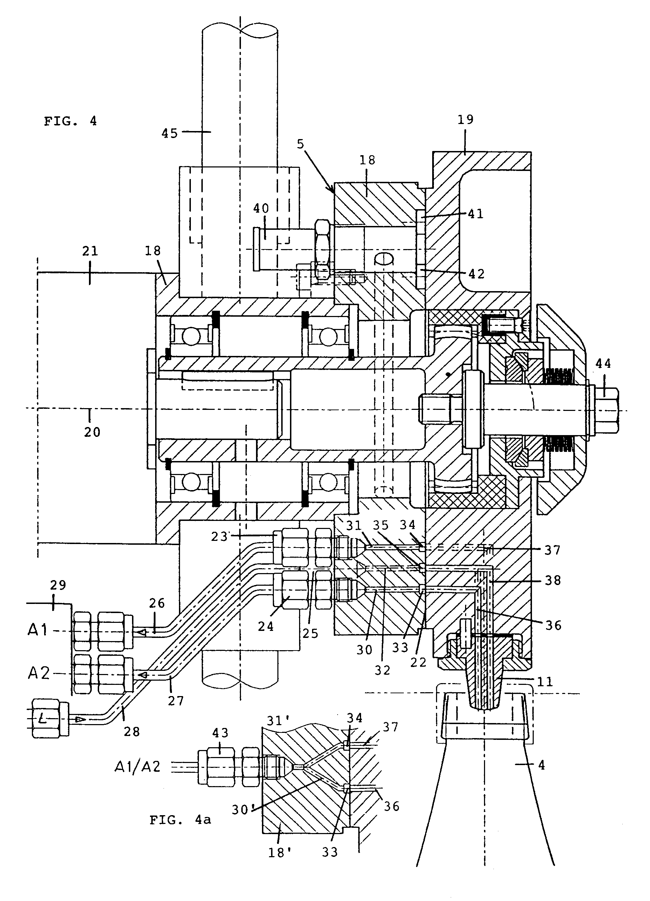

Die Figuren 3 und 4 zeigen den Aufbau eines Probeentnahmeorgans zur Entnahme gasförmiger Proben in genauerer Darstellung. Das Probeentnahmeorgan 5 wird in Figur 3 in einer Frontalansicht mit der Darstellung von im Organ angeordneten Kanälen in gestrichelten Linien und mit der Darstellung einer Zwischenstellung eines der Vorsprünge ebenfalls in gestrichelten Linien gezeigt. Figur 4 zeigt einen Vertikalschnitt. Das Probeentnahmeorgan 5 bildet in dem dargestellten Beispiel zugleich einen Verteilkopf für die Gasproben. Ein Verteilkopf ist bereits aus EP-A-579 952 grundsätzlich bekannt.

Das Probeentnahmeorgan weist in dem gezeigten Beispiel einen feststehenden Teil 18 und einen drehenden Teil 19 auf, welcher um eine zentrale Achse 20 durch einen Motor 21 drehbar ist. Die Drehzahl des Motors ist dabei mit der Geschwindigkeit der Fördereinrichtung synchronisiert. Der feststehende Teil 18 und der drehende Teil 19 berühren sich entlang einer Gleit- und Dichtfläche 22. Am feststehenden Teil 18 sind Anschlüsse 23 und 24 zur Wegführung der den Behältern entnommenen Gasproben vorgesehen, welche mittels Leitungen 26 und 27 zu zwei Analysegeräten A1, A2 geleitet werden die die Gasproben ansaugen; diese Analysegeräte sind nur als Block 29 global dargestellt und werden hier nicht weiter erläutert, da bekannt. Vorzugsweise handelt es sich bei den Analysegeräten um Massenspektrometer oder Detektoren für polyzyklische aromatische Kohlenwasserstoffe (PAK). Ein Anschluss 25 ist weiter am feststehenden Teil 18 vorgesehen, der mit einer Druckluftleitung 28 verbunden ist, damit - wie bekannt - während der Gasprobenentnahme Luft in den Behälter einblasbar ist. Entsprechende Leitungen 30,31,32 für die Gasproben bzw. die Druckluft führen zu kreisbogenabschnittförmigen Kammern 33, 34 und 35 im feststehenden Teil 18, welche mit entsprechenden Leitungen 36, 37 und 38 im beweglichen Teil kommunizieren, wenn diese Leitungen bei der Drehbewegung des drehenden Teils 19 in den Bereich der Kammern 33, 34 und 35 gelangen. Leitungen 36 und 38 sind bei den Vorsprüngen 11, 13 und 15 vorgesehen und münden dort in die Umgebung. Leitungen 37 und 38 sind bei den Vorsprüngen 12, 14 und 16 vorgesehen und münden dort in die Umgebung. Die Vorsprünge, sogenannte Mündungsteile, können je nach Anwendungsfall aus Metall, Kunststoff oder einem Elastomer gefertigt sein und der spezifischen Behältermündung geometrisch angepasst sein, so dass sie die Behältermündung zur Vermeidung von Kontamination nie berühren. Im vorliegenden Beispiel sind sie als feststehende, einfach wechselbare Teile und mit einem Drehsicherungsstift vorgesehen.

Am feststehenden Teil 18 ist ferner ein Anschluss 40 vorgesehen, der mit einer nicht dargestellten Saugpumpe verbunden ist. Der Anschluss 40 ist mit zwei kreisringabschnittsförmigen Kammern 41 und 42 verbunden. Die Vorsprünge 11 bis 16 bzw. die darin vorgesehenen Leitungen 36 und 37 stehen entlang ihres Umlaufes mit diesen Kammern 41 und 42 in Verbindung - mit Ausnahme derjenigen Zeit, da die Verbindung mit den Kammern 33 und 34 erfolgt und die Gasprobe aus dem Behälter entnommen wird - so dass während des Umlaufs der Vorsprünge 11 bis 16 durch diese Umgebungsluft angesaugt und via die Kammern 41 und 42 und den Anschluss 40 zur Saugpumpe gelangt. Dieses Ansaugen von Umgebungsluft dient der Spülung der Vorsprünge 11 bis 16 bzw. der darin angeordneten Leitungen.

Die Funktionsweise zur abwechselnden Zuführung einer Gasprobe zu den Analysegeräten A1 und A2 im Block 29 kann wie folgt beschrieben werden. Der Vorsprung 11 befindet sich in seiner in Figur 3 in ausgezogenen Linien dargestellten Stellung kurz vor dem Eintauchen in den Behälter 4 (ebenfalls mit ausgezogenen Linien dargestellt). Der Kanal 36 des Elementes 11 befindet sich noch in Verbindung mit dem Luft-Ansaugkanal 41 und es wird die oberhalb der Behältermündung angesaugte Luft via den Saugpumpenanschluss 40 zur Saugpumpe geleitet. Der Kanal 38 des Elementes 11 ist bei dieser Stellung des Elementes gerade in Verbindung mit dem Druckluftkanal 35 getreten, so dass aus dem Element 11 nun Druckluft die aus der Druckluftquelle über die Leitung 28 zugeführt wird, austritt. Es beginnt also bereits eine Drucklufteinblasung in den Behälter 4, was dort zu einer besseren Vermischung der im Behälter befindlichen Luft mit zu analysierenden Fremdstoffen führt. Es ist auch möglich, dass dem Probeentnahmeorgan eine separate Einrichtung vorgeschaltet ist, die vorher ein Medium, z.B. Druckluft, in die Behälter einbläst. Bei weiterer Drehbewegung des Organs 5 bzw. des Elementes 11 und bei weiterer Förderbewegung des Behälters 4 taucht das Element 11 in den Behälter 4 ein. Der Kanal 36 des Elementes 11 tritt dabei ausser Verbindung mit der Ansaugkammer 41 und dafür in Verbindung mit der Ansaugkammer 33 bzw. der zugeordneten Leitung 30 und via Leitung 27 mit dem Analysegerät A2, welches somit eine Gasprobe aus dem Behälter zur Analyse ansaugt. Die Leitung 38 des Elementes 11 steht weiterhin in Verbindung mit der Druckluftkammer 35. In Figur 3 ist eine Stellung des Elementes 11 bzw. des Behälters 4 in unterbrochenen Linien dargestellt, welche dem maximalen Eintauchen des Elementes 11 in den Behälter 4 entspricht.

Das Element 16 in Figur 3 hat die Eintauchphase in den zugehörigen Behälter 4' bereits verlassen. Der Kanal 37 des Elementes 16, ist via Kammer 34 und Kanal 31 und Leitung 26 noch in Verbindung mit dem Analysegerät A1 (also nicht mit dem gleichen Analysegerät wie das Element 11). Ebenso wird via Kanal 38 des Elementes 16 und Kanal 32 und die Leitung 28 noch Druckluft ausgeblasen. Bei einer geringfügigen Weiterdrehung des Organs 5 gelangt aber der Kanal 37 des Elementes 16 in Verbindung mit dem Luft-Ansaugkanal 42, so dass die Zufuhr von durch das Element 16 angesaugtem Gas aus dem Behälter 4' zu dem Analysegerät A1 endet.

Der Vorsprung bzw. das Element 12 gelangt als nächstes Element zum Eintauchen in den nächsten (nicht mehr dargestellten) Behälter. Dieses Element 12 weist wiederum einen Kanal 37 auf (wie das Element 16) und kommt daher nach dem Ende der Verbindung mit dem Luft-Ansaugkanal 42 in Verbindung mit dem Gasproben-Ansaugkanal 34 und somit mit dem Analysegerät A1. Das nächste Element 13 hingegen hat einen Kanal 36 (wie das Element 11) und gelangt demzufolge via Kammer 33, Kanal 30 und Leitung 27 in Verbindung mit dem Analysegerät A2. Die sukzessive in die aufeinanderfolgenden Behälter eintauchenden Elemente 11 bis 16 sind also fest je einem der Analysegeräte A1 oder A2 zugeordnet und die Gasproben aus einander folgenden Behältern werden abwechselnd auf die Analysegeräte A1, A2 verteilt.

Figur 4a zeigt eine alternative Ausführungsform der Kanäle 30 und 31 als zu einem einzigen Analysegeräteanschluss 43 führende Kanäle 30' und 31'. Dadurch wird der Betrieb mit nur einem Analysegerät möglich, also z.B. wahlweise das Analysegerät A1, wenn das Analysegerät A2 gewartet oder repariert werden muss, oder generell mit nur einem Analysegerät. Die Aenderung zu nur einem Analysegerät lässt sich relativ einfach durch Ersatz des feststehenden Teiles 18 (Fig. 4) durch das Teil 18' (Figur 4a) erreichen. Eine Auswechslung ist rasch möglich, da das Organ 5 leicht zugänglich ist (im Gegensatz zur bekannten Karussell-Lösung) und durch Lösen der zentralen Befestigung 44 rasch demontiert und montiert werden kann.

Im gezeigten Beispiel erfolgt die Höhenverstellung z.B. mittels einer Spindel 45, welche das Organ 5 mit dem Antriebsmotor 21 in der Höhenlage verstellt.

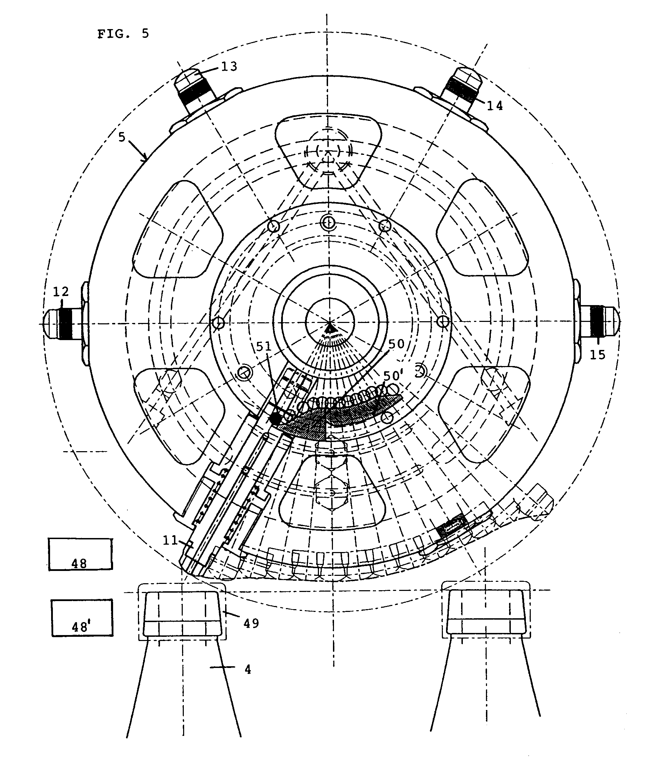

Die Figuren 5, 6 und 7 zeigen ein weiteres Ausführungsbeispiel der Erfindung, wobei das Organ 5 ebenfalls mit sechs Vorsprüngen 11 bis 16 ausgestattet ist und die Zufuhr der Gasproben auf grundsätzlich dieselbe Art und Weise erfolgt, wie bei dem vorbeschriebenen Beispiel. Bei der Ausführungsform nach den Figuren 5 bis 7 wird aber durch eine spezielle Ausgestaltung sichergestellt, dass die Elemente 11 bis 16 nicht in den Behälter eintauchen, wenn dieser noch mit einem Deckel versehen ist (Deckeldetektor 48) oder noch mit einer grossen Menge Flüssigkeit befüllt ist (Flüssigkeitsdetektor 48'), so dass durch die Elemente 11 bis 16 Flüssigkeit angesaugt werden könnte, was die Analysegeräte beschädigen könnte.

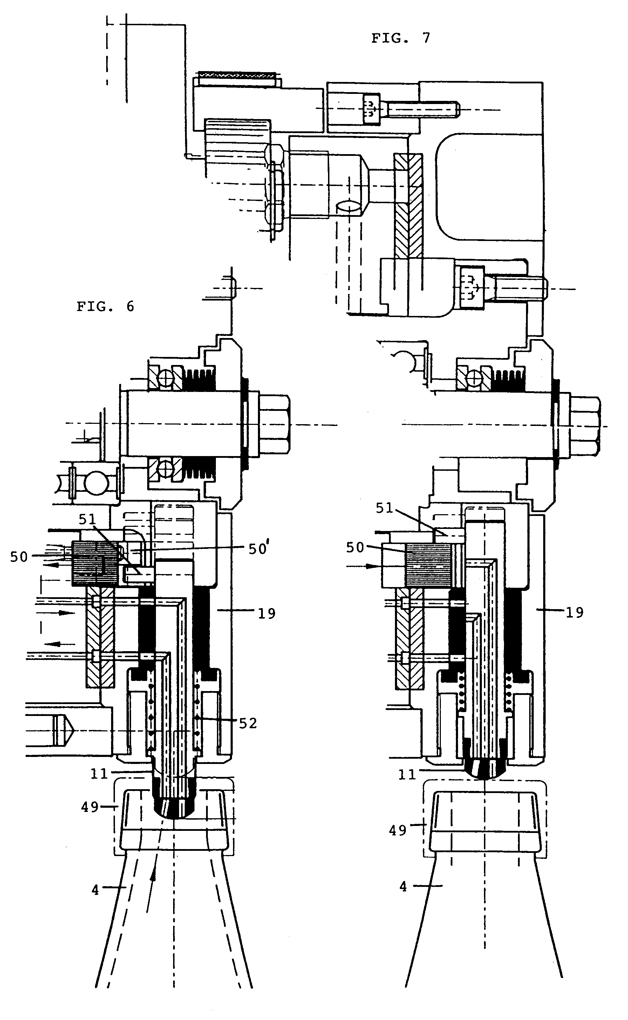

Zu diesem Zweck sind die Elemente 11 bis 16 radial verschieblich im scheibenförmigen Organ 5 angeordnet und es ist eine Rückzugseinrichtung vorgesehen, die ein Zurückziehen des jeweiligen Elementes im Bereich des Behälters ermöglicht. Figur 5 zeigt in ausgezogenen Linien ein Element 11 in Schnittdarstellung in Normalposition kurz vor dem Eintauchen in den Behälter 4. Durch einen Detektor 48 ist indes festgestellt worden, dass der zugehörige Behälter 4 noch mit einem Deckel 49 versehen ist. Durch eine zweiteilige Steuerrampe 50 und 50', welche in Normalstellung (Figur 6) das Element nicht beeinflusst, wird nun bewirkt, dass das Element 11 in radialer Richtung zum Scheibenzentrum hin zurückgezogen wird, so dass das Element den Deckel 49 nicht berührt. Figur 5 zeigt dabei in schwach ausgezogenen Linien eine Reihe von Positionen, die das Element 11 durch die Zurückziehung einnimmt. Am Ende der sonst üblichen Austauchphase wird das Element 11 wieder ausgefahren.

Gemäss Figur 6 ist die Steuerrampe 50 in ihrer Normalstellung soweit zurückgezogen, dass ein Bolzen 51, der an dem im drehbaren Teil 19 verschieblich angeordneten Element 11 befestigt ist, normalerweise nicht an der Steuerrampe aufläuft. Wird die Steuerrampe 50, wie in Figur 7 gezeigt, nach vorne gedrückt, was z.B. elektromechanisch, pneumatisch oder hydraulisch erfolgen kann, so läuft der Bolzen 51 auf der Steuerrampe 50 auf, was ein entsprechendes Zurückziehen des Elementes 11 bewirkt. Am Ende der Steuerrampe 50' (deren Verlauf in Figur 5 ersichtlich ist, in welcher auch verschiedene Positionen des Bolzens 51 dargestellt sind) wird das Element 11 durch die Feder 52 wieder zurück in seine Normalstellung geführt. Die Steuerrampe 50 wird zurückgezogen, sobald der Bolzen 51 auf der fixen Rampe 50' aufgefahren ist, um den nächsten Bolzen rechtzeitig in Normalstellung passieren lassen zu können. Die übrigen Elemente 12 bis 16 sind natürlich ebenfalls so ausgeführt wie das Element 11 und damit zurückziehbar. Bei der Zurückziehung der Elemente wird jeweils der Gasweg unterbrochen, wie dies aus der Figur 7 ersichtlich ist. Die entsprechende, nicht analysierte Flasche wird von der Steuerung der Vorrichtung als mangelhaft eingestuft und aus dem Förderweg ausgeschieden, so wie dies auch mit den als kontaminiert erkannten Behältern geschieht.

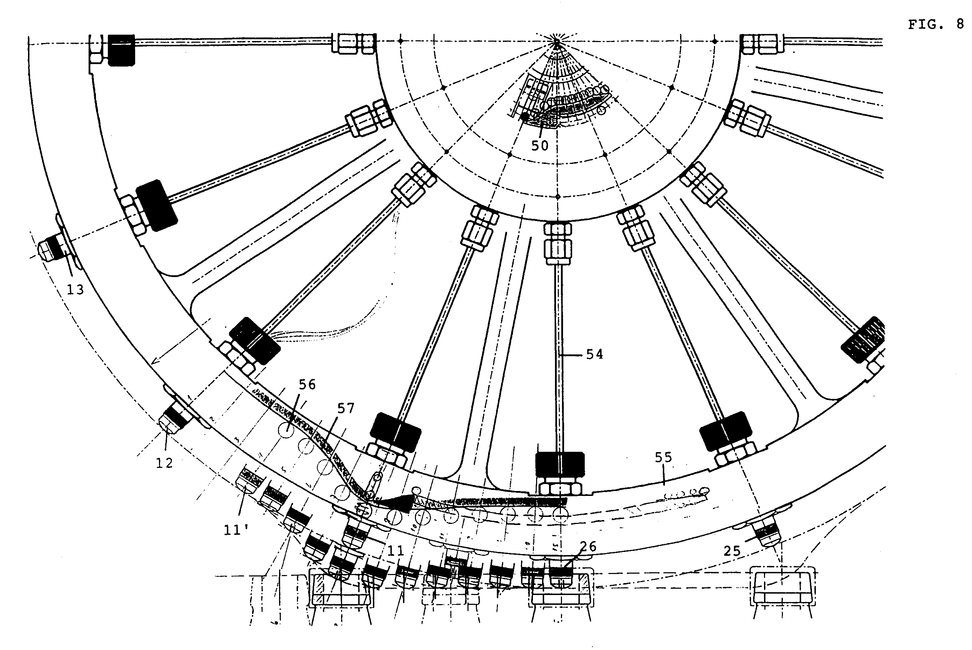

Figur 8 zeigt eine weitere Ausführungsform des Probeentnahmeorgans 5 in teilweiser Darstellung. Dabei ist die innere Scheibe des Probeentnahmeorgans, die grundsätzlich gleich aufgebaut ist, wie anhand der vorhergehenden Beispiele erläutert, um einen radförmigen Kranz erweitert, an dessen Aussenumfang 16 Probeentnahmevorsprünge angeordnet sind, wobei in der Figur nur fünf dieser Vorsprünge 11 bis 13 und 25, 26 ersichtlich sind. Natürlich weist die innere Scheibe entsprechend 16 Kanalanordnungen zur sukzessiven, abwechselnden Durchschaltung der Gasproben an die beiden Analysegeräte auf. Der Vorteil dieser Lösung liegt darin, dass durch den grösseren Aussendurchmesser eine längere Begleitung des Behälters bzw. eine längere Verweildauer des Eintauchelementes im Behälter erzielt wird. Durch die Ausgestaltung mit Scheibe und Kranz muss die Dichtfläche 22 (vgl. Fig. 4) zwischen dem feststehenden und dem beweglichen Teil keinen grösseren Durchmesser aufweisen als bei den vorstehend beschriebenen Beispielen, was herstellungstechnisch bevorzugt ist. Nachteilig an der Lösung mit grösserem Aussendurchmesser ist die Verlängerung der Gaswege, welche durch die Verbindungselemente 54 zwischen dem jeweiligen Vorsprung 11 bis 26 und der Scheibe bedingt sind.

Auch bei dieser Ausführungsform kann ein Zurückziehen der Elemente 11 bis 26 vorgesehen sein, was mit der Steuerrampe 50 angedeutet ist.

In der Figur 8 ist zusätzlich eine weitere Ausführungsform angedeutet, bei welcher im Bereich des Eintauchens ein Vorschieben des Eintauchelementes erfolgt. Zu diesem Zweck ist das Eintauchelement entsprechend verschieblich im Kranz 55 angeordnet und weist einen Bolzen 56 auf, der an einer ortsfesten Steuerkurve 57 aufläuft, so dass sich ein Vorschieben des Elementes ergibt, was in der Figur durch entsprechende Positionen eines Elementes 11' zeichnerisch dargestellt ist. Durch dieses Vorschieben ergibt sich ein sanftes Eintauchen und eine noch erhöhte Verweildauer des Elementes im Behälter.

Allen Ausführungsbeispielen ist der Vorteil gemeinsam, dass sich durch den kontinuierlich ändernden Winkel zwischen Eintauchelement und Behälter auch ein sich ändernder Luft-Einblaswinkel bzw. Absaugwinkel ergibt, was die Durchmischung des Gases mit den kontaminierenden Stoffen und die Absaugung verbessert.

Auch bei der erfindungsgemässen Vorrichtung ist eine Beheizung der Gaswege möglich, wie dies bereits von den Karussell-Vorrichtungen her bekannt ist. Bei den Ausführungen gemäss den Figuren 1 bis 7 ist die Beheizung besonders erleichtert, da die Gaswege zum grössten Teil im feststehenden Teil erfolgen, was die Zuführung des Heizstromes erleichtert (Wegfall von Schleifringstromübertragern). Bei den genannten Ausführungsformen ist der Gasweg zudem ohnehin äusserst kurz, im Vergleich zu den bekannten Karussell-Lösungen. Bei dem Ausführungsbeispiel gemäss Figur 8 ergibt sich ein gegenüber Karussell-Vorrichtungen immer noch deutlich kürzerer Gasweg. Eine Beheizung der Leitungen 54 bedingt hierbei aber eine Stromzuführung zu den Heizwicklungen über Schleifringe.

Die Höhenverstellung des Probeentnahmeorgans 5 ist bereits zur Anpassung an die Behälterhöhe (z.B. 0,5 1, 1,0 1 oder 1,5 1 Flaschen) erwähnt worden. Bei einem aussermittigen Antrieb kann eine entsprechende Spanneinrichtung für den Antriebsriemen oder die Antriebskette vorgesehen sein, so dass die Höhenkorrektur keine spezielle Aenderung der Spannung des Riemens oder der Kette erfordert. Diesfalls unproblematisch ist der zentrale Antrieb, wie in Figur 4 gezeigt.

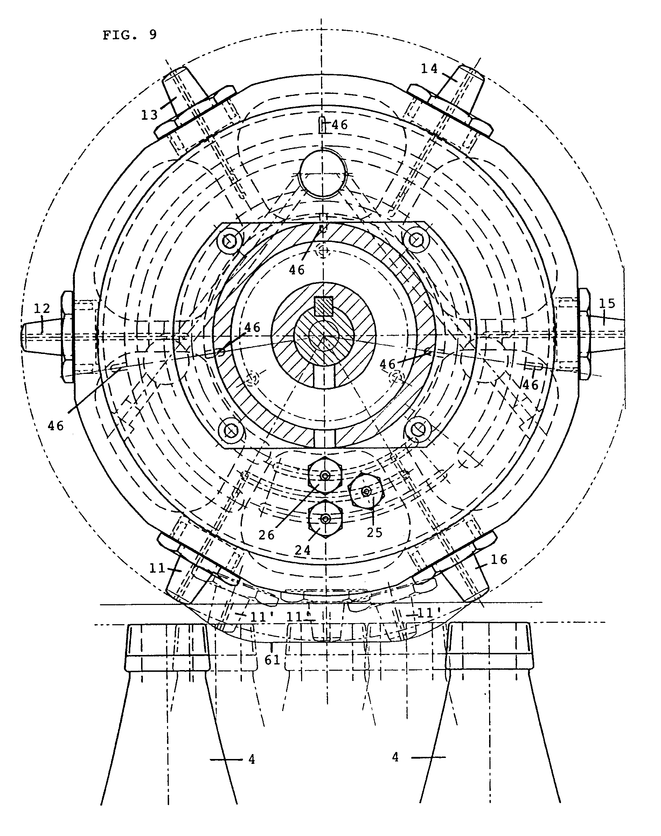

Die Höheneinstellung kann aber auch als dynamische Höhenveränderung mit einer entsprechenden Steuerung und einem schnellen Antrieb (Servomotor, Schrittmotor) ausgeführt sein. Diesfalls entfällt die Notwendigkeit eines Zurückziehens einzelner Eintauchelemente wegen eines Behälterdeckels oder Restflüssigkeit, da in diesem Fall der gesamte Verteilkopf bzw. das Probeentnahmeorgan kurzzeitig nach oben verfahren wird, um das Eintauchen zu verhindern. Vorzugsweise wird auch die Drehgeschwindigkeit angepasst, um bei veränderter Höhe ein synchrones Begleiten des Behälters zu gewährleisten. Eine weitere Ausführungsform der Höhenverstellung beinhaltet eine zusätzliche Absenkungsmöglichkeit des gesamten Organs 5, um die Eintauchtiefe zu erhöhen bzw. die Eintauchzeit zu verlängern. Ein derartiger Effekt ist in Figur 9 gezeigt, wo anhand der Kurve 61 dargestellt ist, wie sich das Absenken des Kopfes auf das Eintauchen der Probeentnahmeorgane auswirkt. In der Figur 9 sind ferner radiale Kanäle 46 dargestellt, die mit dem Sauganschluss 40 verbunden sind. Diese Absaugkanäle 46 dienen der Reinigung der Lauffläche zwischen dem feststehenden Teil 18 und dem beweglichen Teil 19.

Eine weitere Ausführungsform beinhaltet eine Verschiebbarkeit des Organs 5 in Richtung bzw. Gegenrichtung des Förderweges, um eine weitere Bewegungsfreiheit zu erreichen. Entsprechende Koppelkurven-, Exzenter- und x/y-Antriebe sind bekannt und werden hier nicht näher erläutert.

Die Steuerung der Vorrichtung erfolgt auf bekannte Weise durch einen Mikroprozessor oder einen Rechner, durch welche die geschilderten Funktionen mittels entsprechend steuerbarer Antriebe ausführbar sind.

Die bevorzugte Anwendungsart der Vorrichtung liegt bei der Prüfung rücklaufender Mehrweg-Flaschen. Eine Anwendung zur Prüfung anderer Behälter ist aber ohne weiteres möglich.

## Patentansprüche

1. Vorrichtung zur Inspektion von Behältern (4), insbesondere von Mehrweg-Flaschen, auf das Vorhandensein von Verunreinigungen, umfassend eine Fördereinrichtung (1) für die Behälter (4) und mindestens ein jeweils aus einem Behälter eine Probe entnehmendes Probeentnahmeorgan (5), dadurch gekennzeichnet, dass im Inspektionsbereich ein linearer Förderweg (1) zur kontinuierlichen Behälterförderung vorgesehen ist, oberhalb dessen ein parallel zum Förderweg angeordnetes rad- oder scheibenförmiges Probeentnahmeorgan (5) vorgesehen ist, das um eine quer zum Förderweg liegende Achse (20) drehend antreibbar ist.

2. Vorrichtung nach Anspruch 1, dadurch gekennzeichnet, dass das Probeentnahmeorgan (5) entlang seines Umfanges mit mehreren Vorsprüngen (11-16; 11-26) ausgestattet ist, die zum Eintauchen, vorzugsweise zum berührungslosen Eintauchen, in die Behälter (4) bestimmt sind.

3. Vorrichtung nach Anspruch 2, dadurch gekennzeichnet, dass die Vorsprünge radial in Richtung auf das Zentrum des Probeentnahmeorgans verfahrbar sind.

4. Vorrichtung nach Anspruch 3, dadurch gekennzeichnet, dass das Verfahren der Vorsprünge durch Sensoren, insbesondere einen Deckelsensor und/oder einen Flüssigkeitssensor auslösbar ist.

5. Vorrichtung nach Anspruch 3, dadurch gekennzeichnet, dass das Verfahren durch einen Sensor auslösbar ist, der auf Fremdstoffe im Behälter anspricht.

6. Vorrichtung nach einem der Ansprüche 2 bis 5, dadurch gekennzeichnet, dass die Vorsprünge in radialer Richtung vom Zentrum des Probeentnahmeorgans nach aussen verfahrbar sind, um die Eintauchlänge zu verlängern.

7. Vorrichtung nach einem der Ansprüche 1 bis 6, dadurch gekennzeichnet, dass das Probeentnahmeorgan durch einen gesteuerten Antrieb, ausgehend von einer einstellbaren Grundstellung, in seiner Höhe verfahrbar ist.

8. Vorrichtung nach einem der Ansprüche 1 bis 7, dadurch gekennzeichnet, dass das Probeentnahmeorgan durch einen gesteuerten Antrieb in Förderwegrichtung verfahrbar ist.

9. Vorrichtung nach einen der Ansprüche 1 bis 8, dadurch gekennzeichnet, dass das Probeentnahmeorgan als Verteilkopf mit einem feststehenden Teil (18) und einem drehenden Teil (19) ausgestaltet ist und die aus aufeinanderfolgenden Behältern entnommenen Proben abwechselnd auf zwei Analysegeräte (A1,A2) verteilt.

10. Vorrichtung nach Anspruch 9, dadurch gekennzeichnet, dass der Verteilkopf ein auswechselbares Verteilelement (18,18') aufweist, durch welches die Proben zu nur einem Analysegerät (A1 oder A2) zuführbar sind.

11. Vorrichtung nach einem der Ansprüche 9 oder 10, dadurch gekennzeichnet, dass der Verteilkopf Luft-Ansaugkammern (41,42) aufweist, die mit einer Saugpumpe in Verbindung stehen, und dass mittels der Luft-Ansaugkammern während eines Teils des Umlaufes des Probeentnahmeorgans durch dieses Umgebungsluft angesaugt wird.

12. Vorrichtung nach Anspruch 10, dadurch gekennzeichnet, dass an dem Verteilkopf mindestens ein radial verlaufender Kanal (46) vorgesehen ist, der mit der Saugpumpe in Verbindung steht, um die Lauffläche des Verteilkopfes zu reinigen.

13. Vorrichtung nach einem der Ansprüche 9 bis 12, dadurch gekennzeichnet, dass der Verteilkopf einen Anschluss (28) für ein Medium unter Druck aufweist, durch welchen das Medium, vorzugsweise Druckluft, in das Probeentnahmeorgan gelangt und von diesem abgegeben wird.

14. Vorrichtung nach einem der Ansprüche 1 bis 13, dadurch gekennzeichnet, dass dem Probeentnahmeorgan eine Einrichtung vorgeschaltet ist, die ein Medium, vorzugsweise Druckluft, in den Behälter einbringt.

15. Vorrichtung nach einem der Ansprüche 1 bis 14, dadurch gekennzeichnet, dass der Förderweg einen Abschnitt (1) aufweist, in welchem die Behälter in bezug auf das Probeentnahmeorgan positioniert sind, vorzugsweise mittels einer drehend angetriebenen Förderschnecke (9).
